# EUROPEAN PATENT SPECIFICATION

(11) **EP 2 476 451 B1**
(45) Date of publication and mention of the grant of the patent: **19.07.2017**
(21) Application number: 12151145.5
(22) Date of filing: 13.01.2012
(51) Int. Cl.: A61M 5/32, A61M 25/06

(54) **Needle guard**
Nadelschutz
Garde-aiguille

(30) Priority: 17.01.2011 IN 105DE2011
(43) Date of publication of application: 18.07.2012
(73) Proprietor: Poly Medicure Limited, Faridabad, Haryana 121004 (IN)
(72) Inventor: Baid, Rishi, 110 048 New Delhi (IN)
(74) Representative: Thum, Bernhard

(56) References cited:
- US-A1- 2001 039 401
- US-A1- 2002 103 463
- US-A1- 2008 051 724
- US-B1- 6 749 588

## Description

The invention relates to a needle guard for a needle, in particular for a hypodermic needle of a medical apparatus, such as a safety intravenous catheter apparatus, wherein the needle has a needle tip and a needle shaft and defines an axial direction. The needle guard comprises a base portion having a hole the dimension of which is adapted to the needle shaft such that the needle may extend through the hole and move relative to the needle guard from a ready position in which the needle tip is outside the needle guard to a protective position in which the needle tip is covered by the needle guard. The needle guard further includes first and second arms extending distally from the base portion.

It is well-known that blood bone diseases such as AIDS and hepatitis present significant risks to health-care workers administering vascular injections. Accidental needle sticks or needle pricks have become a major concern to health-care workers. The chances of needle stick are increased during an emergency with several aspects required to be handled. Likewise, during disposal, an exposed needle tip may be and usually is a threat to the medical waste handler.

In order to adequately protect health-care workers from inadvertent needle stick and wounding, safety catheter assemblies have been developed to automatically cover and shield the needle tip after its withdrawal from the patient, see for instance document US 6,749,588 B1, in particular figure 49. These assemblies have taken a number of embodiments and have various degrees of elaboration. However, the safety mechanisms implemented in these assemblies increase costs of manufacture substantially and may malfunction, especially in a fluid-filled environment where it may stick or slip. Some of the known needle protecting systems require multiple parts, which drives up the manufacturing cost for a disposable unit.

The cost-benefit requirements of the medical industry call for an inexpensive needle protecting system which is disposable along with the needle. Furthermore, the system must be quick and easy to use as to present as little in position as possible to the administration and function of the safety IV catheter assembly.

Therefore, it is an object of the invention to provide a protective system which is simple and dependable in its deployment, inexpensive to manufacture, expedient in its operation and effective in protecting a needle tip, and which ensures correct functioning even after longer shelf life.

This object is satisfied by a needle guard in accordance with claim 1.

The needle guard of the present invention includes a base portion having a hole the dimension of which is adapted to the needle shaft such that the needle may extend through the hole and move relative to the needle guard from a ready position in which the needle tip is outside the needle guard to a protective position in which the needle tip is covered by the needle guard. The needle guard further includes first and second arms extending from a distal side of the base portion generally in the axial direction, wherein each of the first and second arms has a main portion and a distal end portion. The main portion of the second arm includes two outer arm sections and an inner arm section, wherein the inner arm section is bent away from the first arm and the outer arm sections are bent towards the first arm, thereby defining a passage for the needle, which is axially aligned with the hole in the base portion. In other words, the inner and outer arm sections create a "three leg formation", with two legs being substantially parallel to each other and extending radially inwards towards the first arm and one leg extending radially outwards in an opposing direction.

Generally speaking, the term proximal refers to a region of the device or a location on the device which is closest to, for example, a clinician using the device. In contrast to this, the term distal refers to a region of the device which is farthest from the clinician, for example, the distal region of a needle will be the region of a needle containing the needle tip which is to be inserted e.g. into a patient's vein.

Prior to use, the needle guard is arranged in a chamber of a medical device, such as in the catheter hub of an intravenous catheter apparatus, and positioned near a proximal end of the needle shaft. In this situation, the needle extends completely through the needle guard, thereby supporting the distal end portions of the first and second arms on the needle shaft and deflecting the first and second arms of the needle guard outwards. In this deflected state, the main portion of the second arm of the needle guard can engage with the wall of the catheter hub to retain the needle guard therein.

Following the insertion of the catheter into a patient, the needle is withdrawn from the catheter tube and the needle shaft moves through the needle guard while the needle guard is retained in the catheter hub. Once the needle tip passes the distal end of the needle guard, i.e. such that the needle shaft no longer supports the distal end portions of the arms, a restoring force ensures that the arms of the needle guard are moved back into alignment with the axial direction of the needle guard, so that the needle tip is blocked by the distal end portions of the arms, i.e. the needle tip is prevented from axially projecting out of the needle guard.

Because of the bent shape of the outer arm sections and the needle passage defined thereby, axial alignment of the needle guard with the needle is ensured at all times, i.e. the needle tip is prevented from protruding sideways from the needle guard when the needle has moved to its protective position. It is to be understood that the width of the inner arm section is substantially equal to or slightly larger than the principal outer profile of the needle shaft, such that the needle is allowed to pass through the needle passage.

By moving back into alignment with the axial direction the main portion of the second arm of the needle guard can disengage from the wall of the catheter hub. In addition, an enlargement of the needle shaft can engage with the base portion to allow removal of the needle guard from the catheter hub and to prevent the needle guard from sliding off the distal end of the needle shaft.

According to an embodiment, each of the outer arm sections comprises two subsections extending at an angle, the vertex of which points towards the first arm. Similarly, the inner arm section comprises two subsections extending at an angle, the vertex of which points away from the first arm. The two subsections of each outer arm section thus form a V-shape, whereas the subsections of the inner arm section form an inverted V-shape. The V-like shape of the outer and inner arm sections facilitates the flexing of the arms to and from a biased condition. However, it needs to be understood that the subsections of the inner and outer arm sections do not necessarily need to extend at sharp angles. It also possible, that the subsections of each arm section are connected to each other by a curved or bent portion defining a certain radius.

According to a further embodiment, a proximal end of each of the arm sections terminates at the base portion. Alternatively, it would be possible to provide an intermediate portion between the arm sections and the base portion, which extends generally in the axial direction.

According to another embodiment, the arm sections are defined by two cuts in the second arm, which extend parallel to one another. Thereby, the needle guard is particularly easy to manufacture. If the arm sections are to terminate at the base portion, the two cuts preferably also terminate at the base portion. In order to obtain a sufficient width of the needle passage, the cuts are preferably spaced apart by a distance equal to or slightly larger than the principal diameter of the needle shaft.

According to yet another embodiment the distal end portions of the arms extend towards each other. The distal end portions thus form transverse walls which prevent the needle tip received in the needle guard from protruding from the needle guard at the distal end of the needle guard.

According to a further embodiment, the distal end portions of the arms form an acute angle with the respective main portions of the arms. In other words, the distal end portions of the arms are bent backwards, i.e. towards the base portion.

Preferably, the free end region of each distal end portion is formed in a hook-like manner such that the distal end portions of the first and second arms lock when the needle adopts its protective position. The needle tip received in the needle guard is thus safely captured inside the needle guard and cannot protrude from the needle guard at the distal end thereof.

The locking of the first and second arms can be achieved, for example, if the free end region of the one distal end portion, e.g. of the first arm, is bent towards the base portion and the free end region of the other distal end portion, e.g. of the second arm, is bent away from the base portion.

According to a further embodiment, the first and second arms are biased towards each other such that the first and second arms are spread apart against a restoring force by the distal end portions being supported on the needle shaft when the needle is in its ready position, and snap together when the needle adopts its protective position and the distal end portions move in front of the needle tip.

The needle guard is particularly easy and inexpensive to manufacture, if the needle guard is an integral stamped and bent part.

Preferably, the needle guard comprises a material having elastic properties. For example, the needle guard can be made of a sheet material, in particular, a metal sheet, such as a stainless steel sheet. However, it is to be understood that other flexible strong materials could also be used to form the needle guard. Because of the elastic properties of the material, the needle guard can be easily designed such that the arms snap together when the needle adopts its protective position. Consequently, there is no need for an additional part providing a restoring force, such as an elastic band surrounding the arms.

A needle guard of the above kind can, for example, be used in an intravenous catheter apparatus. The invention therefore also provides a catheter apparatus including a catheter tube, a catheter hub attached to a proximal end of the catheter tube and having a wall defining a chamber, and a needle having a needle tip and a needle shaft. The catheter apparatus further includes a needle guard in accordance with the present invention, which is slidably arranged on the needle shaft and housed in the chamber of the catheter hub.

According to an embodiment, the needle shaft includes an enlargement near the needle tip, a dimension of which is greater than the hole in the base portion of the needle guard. The enlargement can be made by crimping of the needle shaft. However, other ways of forming the enlargement are possible, such as applying additional material to the needle shaft, e.g. by soldering, welding or gluing etc.

The inner profile of the needle can either be reduced in the region of the enlargement, for example, if the enlargement is formed by crimping, or it can be substantially constant throughout the length of the needle, for example, if the enlargement is formed by applying additional material to the needle shaft.

According to a further embodiment, the main portion of the second arm of the needle guard is engaged with the wall of the catheter hub when the needle is in its ready position, thereby retaining the needle guard in the catheter hub, and disengaged from the wall when the needle is in its protective position, thereby allowing removal of the needle guard from the catheter hub.

Further advantageous embodiments of the invention and preferred apparatuses for carrying out the invention as set forth in the subordinate claims and are described in connection with the accompanying drawings.

The present invention will now be explained in more detail in the following with reference to preferred embodiments and to the accompanying drawings in which are shown:
- Fig. 1: a side view of a needle guard of the invention protecting the tip of a needle;
- Fig. 2: a perspective view of the needle guard of Fig. 1;
- Fig. 3: a bottom view of the needle guard of Fig. 1; and
- Fig. 4: a top view of the needle guard of Fig. 1.

The Figures illustrate a needle guard 10 for a needle 12, in particular for a hypodermic needle of a medical apparatus, such as an intravenous catheter apparatus.

The needle 12 has a needle tip 14 and a needle shaft 16. The needle shaft 16 has a principal outer profile and defines an axial direction. Near the needle tip 14 the needle 12 is provided with an enlargement 18, a maximum outer dimension of which is greater than the principal outer profile of the needle shaft 16. The enlargement 18 can, for example, be formed by crimping of the needle shaft 16. However, it is also possible to form the enlargement 18 by adding additional material to the needle shaft 16, for instance, by welding, soldering or gluing.

The needle guard 10 is an integral stamped and bent part made of a sheet material having elastic properties, such as a stainless steel sheet.

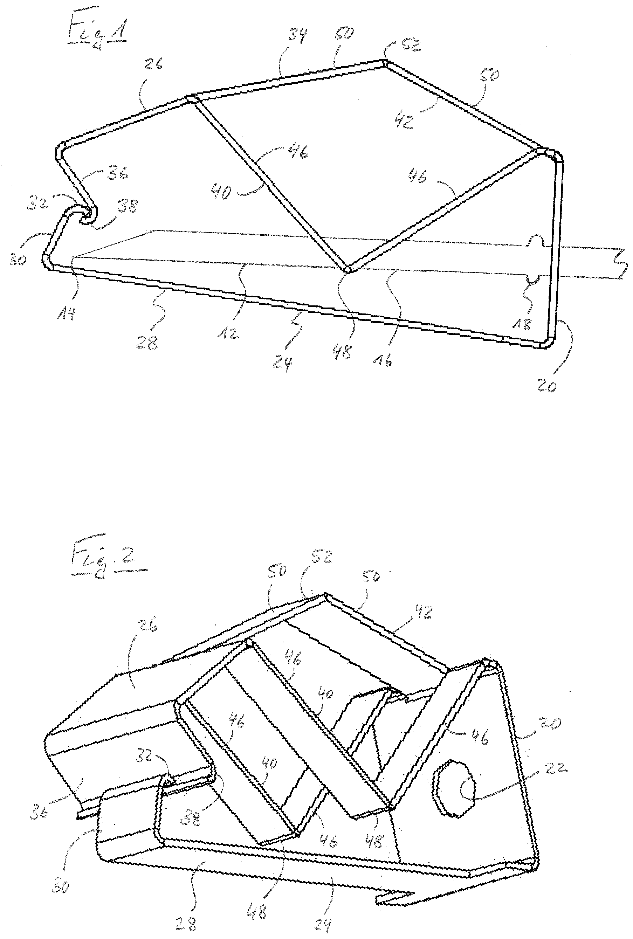

The needle guard 10 comprises a base portion 20 which is provided with a hole 22. The shape of the hole 22 is adapted to the principal outer profile of the needle shaft 16 such that a portion of the needle shaft 16 proximal from the enlargement 18 can extend through the hole 22 and the needle 12 can move relative to the needle guard 10 from a ready position (not shown) in which the needle 12 extends all the way through the needle guard 10 and the needle tip 14 is outside the needle guard 10, to a protective position in which the needle tip 14 is covered by the needle guard 10, as shown in Fig. 1.

A maximum dimension of the hole 22 is smaller than the maximum outer dimension of the enlargement 18 such that the enlargement 18 cannot pass through the hole 22 and the needle guard 10 is prevented from sliding off the needle 12 beyond the needle tip 14.

The needle guard 10 further comprises first and second arms 24, 26 extending distally from the base portion 20 generally in the axial direction on opposite sides of the needle 12.

The first arm 24 has a generally straight main portion 28 and a distal end portion 30 which is bent towards the second arm 26 and which forms an acute angle with the main portion 28, i.e. the distal end portion 30 is bent slightly backwards towards the base portion 20. A free end region 32 of the distal end portion 30 is bent in a hook-like manner towards the base portion 20.

The second arm 26 also has a main portion 34 and a distal end portion 36 which is bent towards the first arm 24 and the base portion 20, such that it forms an acute angle with the main portion 34 of the second arm 26. A free end region 38 of the distal end portion 36 of the second arm 26 is bent in a hook-like manner away from the base portion 20.

When the needle 12 is in its ready position the distal end portions 30, 36 are supported on the needle shaft 16 and the first and second arms 24, 26 are spread apart against a restoring force. When the needle 12 is moved towards its protective position and the needle tip 14 passes the distal end portions 30, 36, the first and second arms 24, 26 snap together and the free end portions 32, 38 engage with each other to lock the first and second arms 24, 26, as shown in Figs. 1 and 2. In this state the distal end portions 30, 36 form a transverse wall in front of the needle tip 14 and prevent the needle tip 14 from distally projecting out of the needle guard 10. At the same time, the enlargement 18 engages with the base portion 20 thereby preventing the needle guard 10 from sliding off the needle 12.

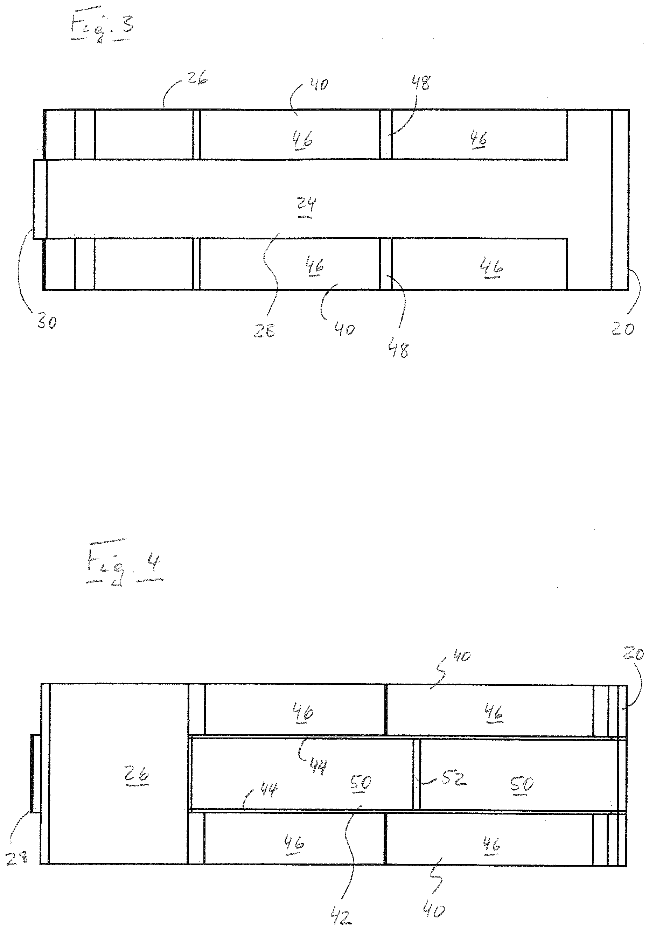

The main portion 34 of the second arm 26 includes two outer arm sections 40 and an inner arm section 42. The outer and inner arm sections 40, 42 are defined by two cuts 44 in the second arm 26, which extend parallel to one another in the axial direction and terminate at the base portion 20 (Fig. 4). The distance between the cuts 44 is equal to or slightly larger than the width of the needle shaft 16.

Each of the outer arm sections 40 comprises two subsections 46 which extend at an angle towards the first arm 24, such that the vertex 48 of the angle points towards the first arm 24.

The bent outer arm sections 40 define a passage for the needle 12 between them, which is axially aligned with the hole 22 in the base portion 20, and thus prevent the needle tip 14 from protruding sideways from the needle guard 10.

The inner arm section 42 comprises two subsections 50 which extend at an angle away from the first arm 26, such that the vertex 52 of the angle points away from the first arm 24.

In the spread apart state of the first and second arms 24, 26, the vertex 52 of the bent inner arm section 42 can engage with a wall of a catheter hub in order to retain needle guard 10 inside the catheter hub as long as the needle tip 14 is not yet received and protected by the needle guard 10.

### List of Numerals

- 10: needle guard
- 12: needle
- 14: needle tip
- 16: needle shaft
- 18: enlargement
- 20: base portion
- 22: hole
- 24: first arm
- 26: second arm
- 28: main portion
- 30: distal end portion
- 32: free end portion
- 34: main portion
- 36: distal end portion
- 38: free end portion
- 40: outer arm section
- 42: inner arm section
- 44: cut
- 46: subsection
- 48: vertex
- 50: subsection
- 52: vertex

## Claims

1. A needle guard (10) for a needle (12), in particular for a hypodermic needle of a medical apparatus, wherein the needle (12) has a needle tip (14), a needle shaft (16) that defines an axial direction along the needle shaft (16) and an enlargement (18) of the needle shaft (16) to prevent the needle guard from sliding off a distal end of the needle shaft (16), the needle guard (10) comprising:
a base portion (20) having a hole (22) the dimensions of which are adapted to the needle shaft (16) such that the needle (12) may extend through the hole (22) and move relative to the needle guard (10) from a ready position in which the needle tip (14) is outside the needle guard (10) to a protective position in which the needle tip (14) is covered by the needle guard (10),
first and second arms (24, 26) extending from a distal side of the base portion (20) generally in the axial direction,
each of the first and second arms (24, 26) having a main portion (28, 34) and a distal end portion (30, 36),
the main portion (34) of the second arm (26) including two outer arm sections (40) extending along the main portion (28) of the first arm (24)and an inner arm section (42) extending along the main portion (28) of the first arm (24) between the outer arm sections (40), wherein the inner arm section (42) is of a shape that is bent away from the main portion (28) of the first arm (24) and the outer arm sections (40) are of a shape that is bent towards the main portion (28) of the first arm (24), wherein the main portion (28) of the first arm (24), the outer arm sections (40) of the main portion (34) of the second arm (26) and the inner arm section (42) of the main portion (34) of the second arm (26) define a passage for the needle (12) between the inner arm section (42) and the main portion (28) of the first arm, and between the outer arm sections (40), which passage is axially aligned with the hole (22) in the base portion (20).

2. The needle guard (10) of claim 1, wherein each of the outer arm sections (40) comprises two subsections (46) extending at an angle the vertex (48) of which points towards the first arm (24), and/or the inner arm section (42) comprises two subsections (50) extending at an angle the vertex (52) of which points away from the first arm (24).

3. The needle guard (10) of claim 1 or 2, wherein a proximal end of each of the arm sections (40, 42) terminates at the base portion (20).

4. The needle guard (10) of any one of the preceding claims, wherein the arm sections (40, 42) are defined by two cuts (44) in the second arm (26), which extend parallel to one another and, in particular, terminate at the base portion (20).

5. The needle guard (10) of any one of the preceding claims, wherein the distal end portions (30, 36) of the first and second arms (24, 26) extend towards each other.

6. The needle guard (10) of any one of the preceding claims, wherein the distal end portions (30, 36) each form an acute angle with their respective main portion (28, 24).

7. The needle guard (10) of any one of the preceding claims, wherein the free end region (32, 38) of each distal end portion (30, 36) is formed in a hook-like manner such that the distal end portions (30, 36) of the first and second arms (24, 26) lock when the needle (12) adopts its protective position.

8. The needle guard (10) of any one of the preceding claims, wherein the free end region (32) of the one distal end portion (30), e.g. of the first arm (24), is bent towards the base portion (20) and the free end region (38) of the other distal end portion (36), e.g. of the second arm (26), is bent away from the base portion (20).

9. The needle guard (10) of any one of the preceding claims, wherein the first and second arms (24, 26) are biased towards each other such that the first and second arms (24, 26) are spread apart against a restoring force by the distal end portions (30, 36) being supported on the needle shaft (16) when the needle (12) is in its ready position, and snap together when the needle (12) adopts its protective position and the distal end portions (30, 36) move in front of the needle tip (14).

10. The needle guard (10) of any one of the preceding claims, wherein the needle guard (10) is an integral stamped and bent part.

11. The needle guard (10) of any one of the preceding claims, wherein the needle guard (10) comprises a material having elastic properties.

12. The needle guard (10) of any one of the preceding claims, wherein the needle guard (10) is made of a sheet material, in particular, a metal sheet, such as a stainless steel sheet.

13. An intravenous catheter apparatus comprising:
a catheter tube;
a catheter hub attached to a proximal end of the catheter tube and having a wall defining a chamber;
a needle (12) having a needle tip (14) and a needle shaft (16); and
a needle guard (10) in accordance with any one of the preceding claims, which is slidably arranged on the needle shaft (16) and housed in the chamber of the catheter hub.

14. The intravenous catheter apparatus of claim 13, wherein the needle shaft (16) includes an enlargement (18) proximal from the needle tip (14), a dimension of which is greater than the hole (22) in the base portion (20) of the needle guard (10).

15. The intravenous catheter apparatus of claim 13 or 14, wherein the main portion (34) of the second arm (26) of the needle guard (10) is engaged with the wall of the catheter hub when the needle (12) is in its ready position and disengaged from the wall when the needle (12) is in its protective position.

## Patentansprüche

1. Nadelschutz (10) für eine Nadel (12), insbesondere für eine hypodermische Nadel einer medizinischen Vorrichtung, wobei die Nadel (12) eine Nadelspitze (14), einen Nadelschaft (16), der eine axiale Richtung entlang dem Nadelschaft (16) definiert und eine Verdickung (18) des Nadelschaftes (16) aufweist, um zu verhindern, dass der Nadelschutz vom distalen Ende des Nadelschaftes (16) rutscht, umfassend:
einen Basisabschnitt (20) mit einer Öffnung (22), deren Abmessungen an den Nadelschaft (16) so angepasst sind, dass sich die Nadel (12) durch die Öffnung (22) erstrecken und sich relativ zum Nadelschutz (10) aus einer Bereitschaftsposition, in der sich die Nadelspitze (14) außerhalb des Nadelschutzes (10) befindet, in eine geschützte Position, in der die Nadelspitze (14) vom Nadelschutz (10) abgedeckt ist, bewegen kann,
einen ersten und einen zweiten Arm (24, 26), die sich von der distalen Seite des Basisabschnitts (20) im wesentlichen in der axialen Richtung erstrecken,
wobei der erste und zweite Arm (24, 26) jeweils einen Hauptabschnitt (28, 34) und einen distalen Endabschnitt (30, 36) aufweisen,
wobei der Hauptabschnitt (34) des zweiten Arms (26) zwei äußere Armabschnitte (40) aufweist, die sich den Hauptabschnitt (28) des ersten Arms (24) entlang erstrecken, und einen inneren Armabschnitt (42), der sich entlang dem Hauptabschnitt (28) des ersten Arms (24) zwischen den äußeren Armabschnitten (40) erstreckt, wobei der innere Armabschnitt (42) eine vom Hauptabschnitt (28) des ersten Arms (24) weg gebogene Form aufweist und die äußeren Armabschnitte (40) eine zum Hauptabschnitt (28) des ersten Arms (24) gebogene Form aufweisen, wobei der Hauptabschnitt (28) des ersten Arms (24), die äußeren Armabschnitte (40) des Hauptabschnitts (34) des zweiten Arms (26) und der innere Armabschnitt (42) des Hauptabschnitts (34) des zweiten Arms (26) einen Durchgang für die Nadel (12) zwischen dem inneren Armabschnitt (42) und dem Hauptabschnitt (28) des erstem Arms und zwischen den äußeren Armabschnitten (40) definieren, wobei der Durchgang axial zu der Öffnung (22) im Basisabschnitt (20) ausgerichtet ist.

2. Nadelschutz (10) nach Anspruch 1, wobei jeder der äußeren Armabschnitte (40) zwei Unterabschnitte (46) aufweist, die sich unter einem Winkel erstrecken, dessen Scheitel (48) zum ersten Arm (24) hinweist, und/oder wobei der innere Armabschnitt (42) zwei Unterabschnitte (50) aufweist, die sich unter einem Winkel erstrecken, dessen Scheitel (52) vom ersten Arm (24) weg weist.

3. Nadelschutz (10) nach Anspruch 1 oder 2, wobei ein proximales Ende jedes der Armabschnitte (40, 42) am Basisabschnitt (20) endet.

4. Nadelschutz (10) nach einem der vorigen Ansprüche, wobei die Armabschnitte (40, 42) durch zwei Schnitte (44) im zweiten Arm (26) definiert sind, die parallel zueinander verlaufen und insbesondere am Basisabschnitt (20) enden.

5. Nadelschutz (10) nach einem der vorigen Ansprüche, wobei sich die distalen Endabschnitte (30, 36) des ersten und zweiten Arms (24, 26) aufeinander zu erstrecken.

6. Nadelschutz (10) nach einem der vorigen Ansprüche, wobei jeder der distalen Endabschnitte (30, 36) einen spitzen Winkel mit dem jeweiligen Hauptabschnitt (28, 24) bildet.

7. Nadelschutz (10) nach einem der vorigen Ansprüche, wobei die freie Endzone (32, 38) jedes distalen Endabschnitts (30, 36) hakenförmig ausgebildet ist, so dass die distalen Endabschnitte (30, 36) des ersten und zweiten Arms (24, 26) einrasten, wenn die Nadel (12) die geschützte Position einnimmt.

8. Nadelschutz (10) nach einem der vorigen Ansprüche, wobei die freie Endzone (32) des einen distalen Endabschnitts (30), z. B. des ersten Arms (24), zum Basisabschnitt (20) gebogen ist und die freie Endzone (38) des anderen distalen Endabschnitts (36), z. B. des zweiten Arms (26), vom Basisabschnitt (20) weg gebogen ist.

9. Nadelschutz (10) nach einem der vorigen Ansprüche, wobei der erste und zweite Arm (24, 26) zueinander so vorgespannt sind, dass der erste und zweite Arm (24, 26) gegen die Rückholkraft der sich am Nadelschaft (16) abstützenden distalen Endabschnitte (30, 36) auseinander gespreizt werden, wenn sich die Nadel (12) in der Bereitschaftsposition befindet, und zusammenklappen, wenn die Nadel (12) ihre geschützte Position einnimmt und sich die distalen Endabschnitte (30, 36) vor die Nadelspitze (14) bewegen.

10. Nadelschutz (10) nach einem der vorigen Ansprüche, wobei der Nadelschutz (10) ein einstückig gestanztes und gebogenes Teil ist.

11. Nadelschutz (10) nach einem der vorigen Ansprüche, wobei der Nadelschutz (10) ein Material mit elastischen Eigenschaften aufweist.

12. Nadelschutz (10) nach einem der vorigen Ansprüche, wobei der Nadelschutz (10) aus einem Blechmaterial besteht, wie aus einem Edelstahlblech.

13. Intravenöses Kathetergerät, aufweisend:
ein Katheterrohr;
eine Katheter-Schraubverbindung am proximalen Ende des Katheterrohrs mit einer eine Kammer definierenden Wand;
eine Nadel (12) mit einer Nadelspitze (14) und einem Nadelschaft (16); und
einen Nadelschutz (10) nach einem der vorigen Ansprüche, der verschieblich auf dem Nadelschaft (16) angeordnet und in der Kammer der Katheter-Schraubverbindung untergebracht ist.

14. Intravenöses Kathetergerät nach Anspruch 13, wobei der Nadelschaft (16) eine proximal von der Nadelspitze (14) befindliche Verdickung (18) enthält, deren Abmessung größer ist als die Öffnung (22) im Basisabschnitt (22) des Nadelschutzes (10).

15. Intravenöses Kathetergerät nach einem der Anspruch 13 oder 14, wobei der Hauptabschnitt (34) des zweiten Arms (26) des Nadelschutzes (10) mit der Wand der Katheter-Schraubverbindung in Eingriff steht, wenn sich die Nadel (12) in der Bereitschaftsposition befindet, und außer Eingriff mit der Wand ist, wenn die Nadel (12) ihre geschützte Position einnimmt.

## Revendications

1. Garde-aiguille (10) pour une aiguille (12), en particulier pour une aiguille hypodermique d'un appareil médical, dans lequel l'aiguille (12) a une pointe (14) d'aiguille, une tige (16) d'aiguille qui définit un sens axial le long de la tige (16) d'aiguille et un élargissement (18) de la tige (16) d'aiguille pour empêcher que le garde-aiguille ne glisse hors d'une extrémité distale de la tige (16) d'aiguille, le garde-aiguille (10) comprenant :
une partie (20) de base ayant un trou (22) dont les dimensions sont adaptées à la tige (16) d'aiguille de telle manière que l'aiguille (12) peut s'étendre à travers le trou (22) et se déplacer par rapport au garde-aiguille (10) d'une position prête dans laquelle la pointe (14) d'aiguille est à l'extérieur du garde-aiguille (10) à une position de protection dans laquelle la pointe (14) d'aiguille est recouverte par le garde-aiguille (10),
des premier et deuxième bras (24, 26) s'étendant depuis un côté distal de la partie (20) de base de façon générale dans le sens axial,
chacun des premier et deuxième bras (24, 26) ayant une partie principale (28, 34) et une partie d'extrémité distale (30, 36),
la partie principale (34) du deuxième bras (26) incluant deux sections extérieures (40) de bras s'étendant le long de la partie principale (28) du premier bras (24) et une section intérieure (42) de bras s'étendant le long de la partie principale (28) du premier bras (24) entre les sections extérieures (40) de bras, dans lequel la section intérieure (42) de bras est d'une forme qui est courbée à l'opposé de la partie principale (28) du premier bras (24) et les sections extérieures (40) de bras sont d'une forme qui est courbée vers la partie principale (28) du premier bras (24), dans lequel la partie principale (28) du premier bras (24), les sections extérieures (40) de bras de la partie principale (34) du deuxième bras (26) et la section intérieure (42) de bras de la partie principale (34) du deuxième bras (26) définissent un passage pour l'aiguille (12) entre la section intérieure (42) de bras et la partie principale (28) du premier bras, et entre les sections extérieures (40) de bras, lequel passage est axialement aligné avec le trou (22) dans la partie (20) de base.

2. Garde-aiguille (10) selon la revendication 1, dans lequel chacune des sections extérieures (40) de bras comprend deux sous-sections (46) s'étendant à un angle dont le sommet (48) pointe vers le premier bras (24), et/ou la section intérieure (42) de bras comprend deux sous-sections (50) s'étendant à un angle dont le sommet (52) pointe à l'opposé du premier bras (24).

3. Garde-aiguille (10) selon la revendication 1 ou 2, dans lequel une extrémité proximale de chacune des sections (40, 42) de bras aboutit au niveau de la partie (20) de base.

4. Garde-aiguille (10) selon l'une quelconque des revendications précédentes, dans lequel les sections (40, 42) de bras sont définies par deux découpes (44) dans le deuxième bras (26), qui s'étendent parallèles l'une à l'autre et, en particulier, aboutissent au niveau de la partie (20) de base.

5. Garde-aiguille (10) selon l'une quelconque des revendications précédentes, dans lequel les parties d'extrémité distale (30, 36) des premier et deuxième bras (24, 26) s'étendent l'une vers l'autre.

6. Garde-aiguille (10) selon l'une quelconque des revendications précédentes, dans lequel les parties d'extrémité distale (30, 36) forment chacune un angle aigu avec leur partie principale (28, 24) respective.

7. Garde-aiguille (10) selon l'une quelconque des revendications précédentes, dans lequel la région d'extrémité libre (32, 38) de chaque partie d'extrémité distale (30, 36) est formée d'une manière de type crochet de telle sorte que les parties d'extrémité distale (30, 36) des premier et deuxième bras (24, 26) se verrouillent lorsque l'aiguille (12) adopte sa position de protection.

8. Garde-aiguille (10) selon l'une quelconque des revendications précédentes, dans lequel la région d'extrémité libre (32) de la partie d'extrémité distale (30), par exemple du premier bras (24), est courbée vers la partie (20) de base et la région d'extrémité libre (38) de l'autre partie d'extrémité distale (36), par exemple du deuxième bras (26), est courbée à l'opposée de la partie (20) de base.

9. Garde-aiguille (10) selon l'une quelconque des revendications précédentes, dans lequel les premier et deuxième bras (24, 26) sont poussés l'un vers l'autre de telle manière que les premier et deuxième bras (24, 26) sont séparés contre une force de restauration par les parties d'extrémité distale (30, 36) étant supportées sur la tige (16) d'aiguille lorsque l'aiguille (12) est dans sa position prête, et s'enclenchent ensemble lorsque l'aiguille (12) adopte sa position de protection et que les parties d'extrémité distale (30, 36) se déplacent en avant de la pointe (14) d'aiguille.

10. Garde-aiguille (10) selon l'une quelconque des revendications précédentes, dans lequel le garde-aiguille (10) est une partie intégrale forgée et courbée.

11. Garde-aiguille (10) selon l'une quelconque des revendications précédentes, dans lequel le garde-aiguille (10) comprend un matériau ayant des propriétés élastiques.

12. Garde-aiguille (10) selon l'une quelconque des revendications précédentes, dans lequel le garde-aiguille (10) est constitué d'un matériau en feuille, en particulier, une feuille de métal, telle qu'une feuille d'acier inoxydable.

13. Appareil de cathéter intraveineux comprenant :
un tube de cathéter ;
une embase de cathéter fixée à une extrémité proximale du tube de cathéter et ayant une paroi définissant une chambre ;
un aiguille (12) ayant une pointe (14) d'aiguille et une tige (16) d'aiguille ; et
un garde-aiguille (10) selon l'une quelconque des revendications précédentes, qui est agencé coulissant sur la tige (16) d'aiguille et logé dans la chambre de l'embase de cathéter.

14. Appareil de cathéter intraveineux selon la revendication 13, dans lequel la tige (16) d'aiguille inclut un élargissement (18) proximal par rapport à la pointe (14) d'aiguille, dont une dimension est supérieure au trou (22) dans la partie (20) de base du garde-aiguille (10).

15. Appareil de cathéter intraveineux selon la revendication 13 ou 14, dans lequel la partie principale (34) du deuxième bras (26) du garde-aiguille (10) est engagée avec la paroi de l'embase de cathéter lorsque l'aiguille (12) est dans sa position prête et désengagée de la paroi lorsque l'aiguille (12) est dans sa position de protection.
